# EUROPÄISCHE PATENTSCHRIFT

(11) **EP 1 515 943 B1**
(45) Veröffentlichungstag und Bekanntmachung des Hinweises auf die Patenterteilung: **30.08.2006**
(21) Anmeldenummer: 03738036.7
(22) Anmeldetag: 14.06.2003
(51) Int. Cl.: C07C 235/34

(54) **VERWENDUNG VON MANDELSÄUREALKYLAMIDEN ALS AROMASTOFFE**
USE OF ALKYLAMIDOMANDELATES AS FLAVOURINGS
UTILISATION D'ALKYLAMIDES D'ACIDE MANDELIQUE COMME AROMATISANTS

(30) Priorität: 17.06.2002 DE 10226942
(43) Veröffentlichungstag der Anmeldung: 23.03.2005
(73) Patentinhaber: Symrise GmbH & Co. KG, 37603 Holzminden (DE)
(72) Erfinder: LEY, Jakob, Peter, 37603 Holzminden (DE); KRAMMER, Gerhard, 37603 Holzminden (DE); MACHINEK, Arnold, 37603 Holzminden (DE)
(74) Vertreter: Eisenführ, Speiser & Partner
(86) Internationale Anmeldenummer: PCT/EP2003/006292
(87) Internationale Veröffentlichungsnummer: WO 2003/106404

(56) Entgegenhaltungen:
- WO-A-01/98258
- WALPOLE C S J ET AL: "ANALOGUES OF CAPSAICIN WITH AGONIST ACTIVITY AS NOVEL ANALGESIC AGENTS;STRUCTURE-ACTIVITY STUDIES. 2. THE AMIDE BOND B-REGION" JOURNAL OF MEDICINAL CHEMISTRY, AMERICAN CHEMICAL SOCIETY. WASHINGTON, US, Bd. 36, Nr. 16, 1993, Seiten 2373-2380, XP001145824 ISSN: 0022-2623 in der Anmeldung erwähnt

## Beschreibung

Die Erfindung betrifft die Verwendung von Mandelsäurealkylamiden als Aromastoffe, insbesondere als Scharfstoffe und Aromastoffe mit einem wärmeerzeugenden Effekt, in der Ernährung, der Mundhygiene oder dem Genuss dienenden Zubereitungen. Ferner betrifft die Erfindung der Ernährung, der Mundhygiene oder dem Genuss dienende Zubereitungen, enthaltend die erfindungsgemäßen Mandelsäurealkylamide sowie Verfahren zur Herstellung der erfindungsgemäßen Zubereitungen.

Capsaicin [*N*-(4-Hydroxy-3-methoxybenzyl)-8-methyl-(6*E*)-nonensäureamid, vgl. Struktur 1, Abbildung 1] und andere Capsaicinoide sind als scharf schmeckende und wärmeerzeugende Aromastoffe aus verschiedenen Capsicum-Arten, insbesondere Chili, schon seit 1871 bekannt. Bei entsprechend geringer Dosierung der Capsaicinoide (der Schwellenwert liegt bei einer Verdünnung von ca. 1:10⁵) wird nur eine angenehme, neutrale Schärfe und ein Wärmegefühl im Mund wahrgenommen. Problematisch ist bei Capsaicin die hohe akute Toxizität (LD₅₀ (Maus oral) 47 mg), die die Anwendbarkeit bei der Zubereitung erschwert, sowie die bei häufiger Anwendung und Überdosierung auftretende chron. Gastritis, Nieren- und Leberschädigung (Römpp Lexikon Naturstoffchemie, Thieme 1997, S. 109). Somit besteht trotz der guten sensorischen Eigenschaften ein Bedarf an weniger problematischen Scharfstoffen. Das im weißen Pfeffer vorkommende Piperin (1-Piperoylpiperidin, vgl. Struktur 2, Abbildung 1) verursacht zwar auch einen starken scharfen Eindruck (Römpp Lexikon Naturstoffchemie, Thieme 1997, S. 500), zeigt aber im Vergleich zu Capsaicin eine relative Schärfe von nur ca. 1 %. Darüber hinaus besitzt Piperin einen intensiven Eigengeschmack, der an Pfeffer erinnert, so dass die Anwendung in vielen Zubereitungen nur beschränkt erfolgen kann.

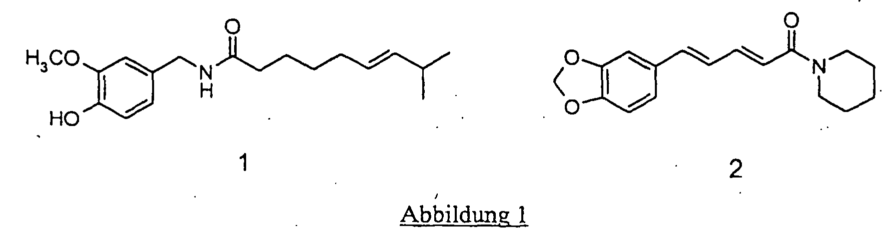

Aufgabe der vorliegenden Erfindung war es, Stoffe mit einem scharfen und/oder wärmeerzeugenden Effekt sowie einem neutralen Aromaprofil zu finden und als Aromastoffe in der Ernährung, der Mundhygiene oder dem Genuss dienenden Zubereitungen verwendet werden können.

Die Erfindung betrifft daher die Verwendung von Mandelsäurealkylamiden der allgemeinen Formel (I) 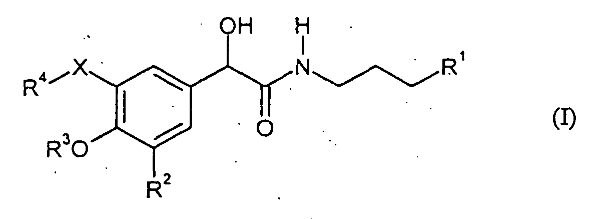 wobei
- X: eine Einfachbindung oder ein Sauerstoffatom darstellt
und
- R¹: einen linearen oder verzweigten Alkylrest mit 1 bis 20 Kohlenstoffatomen oder einen linearen oder verzweigten Alkenylrest mit 2 bis 20 Kohlenstoffatomen darstellt
und
- R²: ein Wasserstoffatom, eine Hydroxygruppe oder eine Gruppe O-R⁵ darstellt
und
- R³, R⁴ und R⁵: unabhängig voneinander Wasserstoff oder einen Niederalkylrest oder einen Niederalkenylrest darstellt
oder
R³ und R⁴ zusammen eine Gruppe -CR⁶R⁷- darstellt
und R⁶ und R⁷ unabhängig voneinander Wasserstoff oder Niederalkylreste oder Niederalkenylreste darstellen,
und deren verschiedenen Stereoisomeren oder deren Gemische als Aromastoffe, bevorzugt als Scharfstoffe oder Aromastoffe mit einem wärmeerzeugenden Effekt, insbesondere bevorzugt als Scharfstoffe oder Aromastoffe mit einem wärmeerzeugenden Effekt in der Ernährung, der Mundhygiene oder dem Genuss dienenden Zubereitungen. Unter wärmeerzeugenden Stoffen bzw. Stoffen mit einem wärmeerzeugenden Effekt werden solche verstanden, die sensorisch einen Wärmeeindruck hervorrufen.

Ein Niederalkylrest besteht aus 1 bis 5 Kohlenstoffatomen und kann beispielsweise sein: Methyl, Ethyl, 1-Propyl, 2-Propyl-, 1-Butyl, 2-Butyl, tert.-Butyl, 2-Methylprop-1-yl, 1-, 2- oder 3-Pentyl-, 2-Methylbut-1-yl, 2-Methylbut-2-yl, 3-Methylbut-1-yl oder 3-Methylbut-2-yl.

Ein Niederalkenylrest besteht aus 2 bis 5 Kohlenstoffatomen und kann beispielsweise sein: Ethenyl, Prop-2-en-1-yl, Prop-1-en-1-yl, Prop-1-en-2-yl, 1- oder 2-Cyclopropenyl-, But-1-en-1-yl, But-1-en-2-yl, But-1-en-3-yl, But-2-en-1-yl, But-3-en-1-yl, But-2-en-2-yl, 2-Methylprop-1-en-1-yl, 2-Methylprop-2-en-1-yl, 1,3-Butadien-1-yl, 1,3-Butadien-2-yl, Pent-1-en-1-yl, Pent-1-en-2-yl, Pent-1-en-3-yl, Pent-1-en-4-yl, Pent-2-en-1-yl, Pent-2-en-2-yl, Pent-2-en-3-yl, Pent-2-en-4-yl, Pent-3-en-1-yl, Pent-4-en-1-yl, 1,3-Pentadien-1-yl, 1,3-Pentadien-2-yl, 1,3-Pentadien-3-yl, 2,4-Pentadien-2-yl, 2,4-Pentadien-1-yl, 1,4-Pentadien-1-yl, 1,4-Pentadien-2-yl, 1,4-Pentadien-3-yl, 1-,2- oder 3-Cyclopentenyl, 1-,2- oder 3-Cyclopentadienyl, 3-Methylbut-1-en-1-yl, 3-Methylbut-1-en-2-yl, 3-Methylbut-1-en-3-yl, 3-Methylbut-1-en-4-yl, 3-Methylbut-2-en-1-yl, 3-Methylbut-2-en-2-yl, 3-Methylbut-2-en-4-yl, 2-Methylbut-1-en-1-yl, 2-Methylbut-1-en-3-yl, 2-Methylbut-1-en-4-yl, 2-Methylidenbut-1-yl, 2-Methyl-1,3-butadien-1-yl, 2-Methyl-1,3-butadien-3-yl, 2-Methyl-1,3-butadien-4-yl, 2-Methylidenbut-3-en-1-yl, und die jeweiligen gegebenenfalls möglichen Z- und E-Isomere der vorgenannten Reste.

Ein linearer oder verzweigter Alkylrest besteht aus 1 bis 20 Kohlenstoffatomen und kann beispielsweise sein: Methyl, Ethyl, 1-Propyl, 2-Propyl-, 1-Butyl, 2-Butyl, tert.-Butyl, 2-Methylprop-1-yl, 1-, 2- oder 3-Pentyl-, 2-Methylbuf-1-yl, 2-Methylbut-2-yl, 3-Methylbut-1-yl oder 3-Methylbut-2-yl, 1-, 2- oder 3-Hexyl, 1-, 2-, 3- oder 4-Heptyl-, 1-, 2-, 3- oder 4-Octyl, 1-, 2-, 3- oder 5-Nonyl, oder 1-, 2-, 3-, 4- oder 5-Decyl oder die verschiedenen Isomere von Undecyl, Dodecyl, Tridecyl, Tetradecyl, Pentadecyl, Hexadecyl, Heptadecyl, Octadecyl, Nonadecyl, Eicosyl.

Ein linearer oder verzweigter Alkenylrest besteht aus 2 bis 20 Kohlenstoffatomen und kann beispielsweise sein: Ethenyl, Prop-2-en-1-yl, Prop-1-en-1-yl, Prop-1-en-2-yl, But-1-en-1-yl, But-1-en-2-yl, But-1-en-3-yl, But-2-en-1-yl, But-3-en-1-yl, But-2-en-2-yl, 2-Methylprop-1-en-1-yl, 2-Methylprop-2-en-1-yl, 1,3-Butadien-1-yl, 1,3-Butadien-2-yl, Pent-1-en-1-yl, Pent-1-en-2-yl, Pent-1-en-3-yl, Pent-1-en-4-yl, Pent-2-en-1-yl, Pent-2-en-2-yl, Pent-2-en-3-yl, Pent-2-en-4-yl, Pent-3-en-1-yl, Pent-4-en-1-yl, 1,3-Pentadien-1-yl, 1,3-Pentadien-2-yl, 1,3-Pentadien-3-yl, 2,4-Pentadien-2-yl, 2,4-Pentadien-1-yl, 1,4-Pentadien-1-yl, 1,4-Pentadien-2-yl, 1,4-Pentadien-3-yl, 3-Methylbut-1-en-1-yl, 3-Methylbut-1-en-2-yl, 3-Methylbut-1-en-3-yl, 3-Methylbut-1-en-4-yl, 3-Methylbut-2-en-1-yl, 3-Methylbut-2-en-2-yl, 3-Methylbut-2-en-4-yl, 2-Methylbut-1-en-1-yl, 2-Methylbut-1-en-3-yl, 2-Methylbut-1-en-4-yl, 2-Methylidenbut-1-yl, 2-Methyl-1,3-butadien-1-yl, 2-Methyl-1,3-butadien-3-yl, 2-Methyl-1,3-butadien-4-yl, 2-Methylidenbut-3-en-1-yl, sowie alle möglichen Alkene, Alkadiene, Alkatriene, Alkatetraene und Alkapentaene von C₆ bis C₂₀ und die jeweiligen gegebenenfalls möglichen Z- und E-Isomere der vorgenannten Reste.

Überraschenderweise zeigen die erfindungsgemäßen Mandelsäurealkylamide bei der sensorischen Untersuchung einen angenehmen, starken scharfen und warmen Geschmackseindruck, der neutral ist und relativ lang anhält.

Ein weiterer Gegenstand der vorliegenden Erfindung sind Zubereitungen, Halfertigwaren und Riech-, Aroma- und Geschmackstoffkompositionen, enthaltend diese Verbindungen.

Selbstverständlich können die erfindungsgemäßen Mandelsäurealkylamide auch in kosmetischen oder dermatologischen Zubereitungen zur Wärmeerzeugung auf der Haut verwendet werden.

Besonders bevorzugt ist die Verwendung von Mandelsäurealkylamiden der allgemeinen Formel (I) 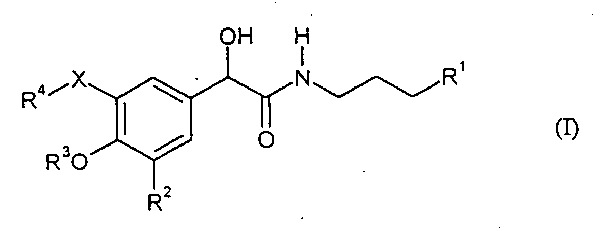 wobei
- R¹: einen linearen oder verzweigten Alkylrest mit 1 bis 10 Kohlenstoffatomen oder einen linearen oder verzweigten Alkenylrest mit 2 bis 10 Kohlenstoffatomen darstellt
und
- R²: ein Wasserstoffatom darstellt,
und
- X: eine Einfachbindung, R³ eine Methylgruppe und R⁴ Wasserstoff darstellen (4-Methoxymandelsäurealkylamide)
oder
- X: ein Sauerstoffatom und R³ Wasserstoff und R⁴ Methyl darstellt (Vanillomandelsäurealkylamide),
oder
- X: ein Sauerstoffatom und R⁴ Wasserstoff und R³ Methyl darstellt (Isovanillomandelsäurealkylamide),
und deren verschiedenen Stereoisomeren oder deren Gemische als Aromastoffe, bevorzugt als Scharfstoffe oder Aromastoffe mit einem wärmeerzeugenden Effekt, insbesondere bevorzugt als Scharfstoffe oder Aromastoffe mit einem wärmeerzeugenden Effekt in der Ernährung, der Mundhygiene oder dem Genuss dienenden Zubereitungen.

Insbesondere bevorzugt ist die Verwendung der Verbindungen
2-(4-Methoxyphenyl)-2-hydroxy-N-heptylessigsäureamid,
2-(4-Methoxyphenyl)-2-hydroxy-N-octylessigsäureamid,
2-(4-Methoxyphenyl)-2-hydroxy-N-nonylessigsäureamid,
2-(3-Hydroxy-4-methoxyphenyl)-2-hydroxy-N-heptylessigsäureamid,
2-(3-Hydroxy-4-methoxyphenyl)-2-hydroxy-N-octylessigsäureamid,
2-(3-Hydroxy-4-methoxyphenyl)-2-hydroxy-N-nonylessigsäureamid,
2-(4-Hydroxy-3-methoxyphenyl)-2-hydroxy-N-heptylessigsäureamid,
2-(4-Hydroxy-3-methoxyphenyl)-2-hydroxy-N-octylessigsäureamid,
2-(4-Hydroxy-3-methoxyphenyl)-2-hydroxy-N-nonylessigsäureamid,
oder
2-(4-Hydroxy-3-methoxyphenyl)-2-hydroxy-N-(7-methyl-1-octyl)essigsäureamid
als Aromastoffe, bevorzugt als Scharfstoffe und Aromastoffe mit einem wärmeerzeugenden Effekt, insbesondere bevorzugt als Scharfstoffe und Aromastoffe mit einem wärmeerzeugenden Effekt in der Ernährung, der Mundhygiene oder dem Genuss dienenden Zubereitungen.

Selbstverständlich können die verschiedenen erfindungsgemäßen Mandelsäurealkylamide, deren Stereoisomere und Salze jeweils alleine oder als Gemische erfindungsgemäß verwendet werden.

Die Salze der erfindungsgemäßen Mandelsäurealkylamide können als ein- oder gegebenenfalls mehrwertige phenolatische Salze mit anorganischen Kationen vorliegen. Bevorzugt sind die Kationen von Lithium, Natrium, Kalium, das Ammoniumion, die Kationen von Magnesium, Calcium und Strontium oder die Kationen von Aluminium, Zink, Kupfer, Eisen oder Mangan.

Das 2-(4-Hydroxy-3-methoxyphenyl)-2-hydroxy-N-octylessigsäureamid (Vanillomandelsäureoctylamid) wurde als Analgeticum in J. Med. Chem., Band 36, Jhrg. 1993, Seiten 2373ff. beschrieben. Eine sensorische Beurteilung der Substanz oder eine Beschreibung einer Verwendung sind aber nicht beschrieben worden. Weitere langkettige 4-Hydroxymandelsäurealkylamide, Vanillomandelsäurealkylamide und Isovanillinsäurealkylamide im Sinne der Erfindung sind nicht bekannt. 3,4-Dihydroxymandelsäurealkylamide wurden in DE-A 100 30 880 als Antioxidantien beschrieben.

In der Literatur wurde insbesondere nicht die Verwendung der erfindungsgemäßen Verbindungen als Aromastoffe oder deren Verwendung als scharf schmeckende Aromastoffe bzw. als Stoffe mit einem wärmeerzeugenden Effekt beschrieben.

### Mandelsäurealkylamide der allgemeinen Formel (I)

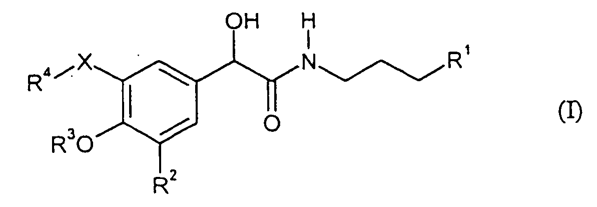

wobei
- R¹: einen linearen oder verzweigten Alkylrest mit 1 bis 20 Kohlenstoffatomen oder einen linearen oder verzweigten Alkenylrest mit 2 bis 20 Kohlenstoffatomen darstellt und
- R²: ein Wasserstoffatom darstellt,
und
entweder
- X: eine Einfachbindung,
- R³: einen Niederalkylrest oder einen Niederalkenylrest und
- R⁴: Wasserstoff darstellen
oder
- X: ein Sauerstoffatom,
- R³: Wasserstoff und
- R⁴: einen Niederalkylrest oder einen Niederalkenylrest darstellen
oder
- X: ein Sauerstoffatom,
- R³: einen Niederalkylrest oder einen Niederalkenylrest und
- R⁴: Wasserstoff darstellen
und deren verschiedenen Stereoisomeren oder deren Gemische mit der Ausnahme, dass X ein Sauerstoffatom, R¹ 1-Pentyl, R² und R³ Wasserstoff und R⁴ .Methyl darstellen, sind neu.
Insbesondere bevorzugt sind Mandelsäurealkylamide der allgemeinen Formel (I),
wobei
- R¹: einen linearen oder verzweigten Alkylrest mit 1 bis 10 Kohlenstoffatomen oder einen linearen oder verzweigten Alkenylrest mit 2 bis 10 Kohlenstoffatomen darstellt mit der Ausnahme, dass R¹ 1-Pentyl darstellt,
und
- R²: ein Wasserstoffatom darstellt,
und
entweder
- X: eine Einfachbindung,
- R³: ein Wasserstoffatom oder eine Methylgruppe und
- R⁴: ein Wasserstoffatom darstellen
oder
- X: ein Sauerstoffatom,
- R³: Wasserstoff und
- R⁴: einen Methylrest darstellen
oder
- X: ein Sauerstoffatom,
- R³: einen Methyl und
- R⁴: Wasserstoff darstellen
und deren verschiedenen Stereoisomeren oder deren Gemische, mit der Ausnahme, dass X ein Sauerstoffatom, R¹ 1-Pentyl, R² und R³ Wasserstoff und R⁴ Methyl darstellen.

Insbesondere bevorzugt sind die Verbindungen
2-(4-Methoxyphenyl)-2-hydroxy-N-heptylessigsäureamid,
2-(4-Methoxyphenyl)-2-hydroxy-N-octylessigsäureamid,
2-(4-Methoxyphenyl)-2-hydroxy-N-nonylessigsäureamid,
2-(3-Hydroxy-4-methoxyphenyl)-2-hydroxy-N-heptylessigsäureamid,
2*-*(3-Hydroxy-y-methoxyphenyl)-z-hydroxy-N-octylessigsäureamid
2-(3-Hydroxy-4-methoxyphenyl)-2-hydroxy-N-nonylessigsäureamid
2-(4-Hydroxy-3-methoxyphenyl)-2-hydroxy-N-heptylessigsäureamid,
2-(4-Hydroxy-3-methoxyphenyl)-2-hydroxy-N-nonylessigsäureamid,
und
2-(4-Hydroxy-3-methoxyphenyl)-2-hydroxy-N-(7-methyl-1-octyl)essigsäureamid.

Die erfindungsgemäßen Mandelsäurealkylarmide können hergestellt werden, dergestalt, dass man eine Mandelsäure der allgemeinen Formein II 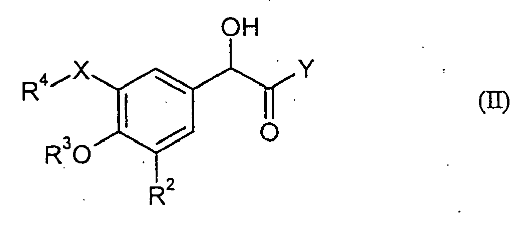 wobei
X, R², R³ und R⁴ die oben genannte Bedeutung haben,
und
- Y: eine aktivierte Abgangsgruppe darstellt,
oder Derivaten, deren OH-Gruppen mit Schutzgruppen geschützt sind, mit einem Alkylamin der allgemeinen Formel (IIIa) 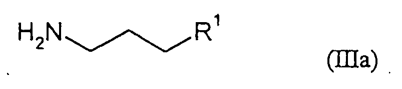 oder einem Alkylammoniumsalz der allgemeinen Formel (IIIb) 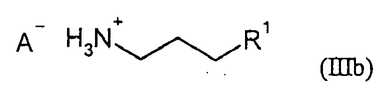 wobei
- R¹: die oben angeführte Bedeutung hat und
- A⁻: ein anorganisches oder organisches Anion, beispielsweise Halogenid, Sulfat, Hydrogensulfat oder Acetat bedeutet,
gegebenenfalls in Gegenwart von Lösemitteln und Hilfsbasen umsetzt und gegebenenfalls die Schutzgruppen der OH-Gruppen abspaltet.

Aktivierte Abgangsgruppen Y sind z.B. die Halogenide, bevorzugt Chlorid, O-Acylreste, bevorzugt O-Acetyl, O-Oxalyl oder ein Rest der allgemeinen Formel II mit Y als Sauerstoff, wobei man symmetrische oder gemischte Säureanhydride erhält, oder Reste O-R⁶, wobei R⁶ Niederalkylreste, gegebenenfalls substituierte Phenole, bevorzugt Mono-, Di- oder Trinitrosubstituierte Phenole, stickstoffhaltige N-Hydroxyheterocyclen, bevorzugt N-Hydroxysuccinimid, N-Hydroxyphthalimid oder N-Hydroxybenzotriazol darstellen.

Als Schutzgruppen verwendet man bevorzugt Acyl-, Carbamat- oder Ethergruppen, z.B. Acetyl-, Benzoyl-, Methoxycarbonyl-, Allyloxycarbonyl-, Methoxymethyl-, Methoxyethoxymethyl-, tert.-Butoxycarbonyl-, Allyl- oder Benzylgruppen.

Als Lösungsmittel können protische oder nicht protische polare und unpolare Lösungsmittel verwendet werden, bevorzugt Wasser, Ketone, Alkohole, Alkylester aliphatischer Säuren, chlorierte Kohlenwasserstoffe, Ether, N-Methylamide. Besonders bevorzugt sind Wasser, Ethanol, Methanol, Aceton, 1,4-Dioxan, N-Methylpyrrolidon, N,N-Dimethylformamid, Tetrahydrofuran, Essigsäureethylester, Chloroform oder auch Gemische der letztgenannten Lösungsmittel.

Als Hilfsbasen können z.B. Ammonium-, Alkalimetall- oder Erdalkalimetallcarbonate, -hydrogencarbonate, -hydroxide, tertiäre aliphatische Amine, z.B. Triethylamin, und anorganische oder organische basische Ionenaustauscher verwendet werden.

Die erfindungsgemäßen Mandelsäurealkylamide werden besonders bevorzugt aus gegebenenfalls an den Hydroxygruppen mit Acetyl- oder Methoxycarbonylgruppen blockierten Mandelsäure-N-hydroxysuccinimidylestern mit Alkylaminen der Formel IIIa oder den Alkylammoniumsalzen der Formel IIIb in einem wasserhaltigen Lösungsmittelgemisch, bevorzugt einem Wasser/1,4-Dioxan- oder Wasser/Aceton-Gemisch mit einer der oben genannten Hilfsbasen bei 5 bis 100°C hergestellt. Vorteilhafterweise werden die Mandelsäure-N-hydroxysuccinimidylester aus der entsprechenden freien Säure und *N*-Hydroxysuccinimid (NHOSu) mittels eines Carbodiimids, vorzugsweise *N,N'*-Dicyclohexylcarbodiimid (DCC) oder Diisopropylcarbodiimid (DIIC), in einem aprotischen Lösungsmittel, vorzugsweise 1,4-Dioxan, Diethylether, tert.-Butylmethylether, Essigsäureethylester oder Tetrahydrofuran, bei 0 bis 50°C, vorzugsweise bei 5 bis 30°C, dargestellt, das gelöste Rohprodukt durch Filtration vom Rückstand getrennt und das Filtrat direkt im Sinne der Erfindung mit dem in Wasser oder Wasser/1,4-Dioxan- oder Wasser/Aceton-Gemisch vorgelegten Alkylaminen der Formel IIIa oder den Alkylammoniumsalzen der Formel IIIb und einer der oben genannten Hilfsbasen umgesetzt. Das Verfahren wird durch das folgende Schema am Beispiel 2-(4-Hydroxy-3-methoxyphenyl)-*N* nonyl-2-hydroxyessigsäureamid verdeutlicht:

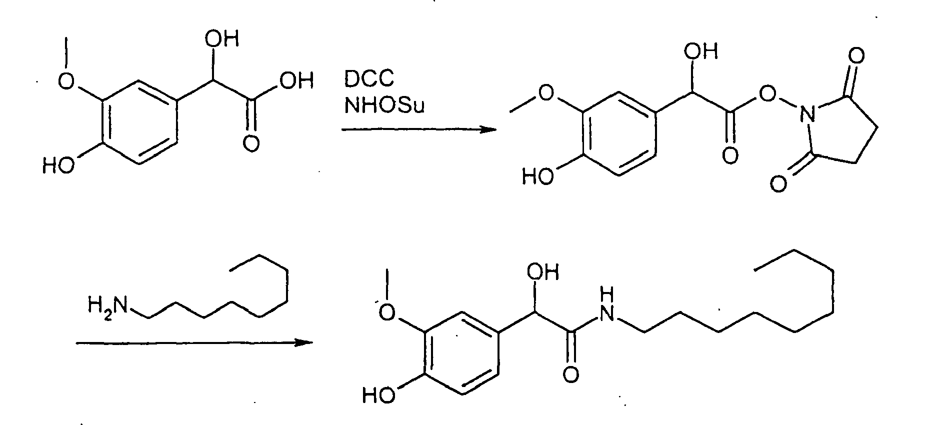

Als Alkylamine werden insbesondere n-Heptylamin, *n*-Octylamin, n-Nonylamin, 7-Methyloctylamin oder deren jeweiligen Ainmoniumsalze verwendet.

Als Mandelsäuren werden bevorzugt 4-Hydroxymandelsäure, 4-Methoxymandelsäure, 4-Hydroxy-3-methoxymandelsäure oder 3-Hydroxy-4-methoxymandelsäuren, deren Stereoisomeren sowie deren Gemische verwendet.

Die erfindungsgemäßen Mandelsäurealkylamide können aber auch durch direkte Kondensation der freien Säuren der allgemeinen Formel II,
wobei X, R², R³ und R⁴ die oben genannten Bedeutungen haben,
und Y Hydroxy darstellt,
mit einem Alkylamin der allgemeinen Formel IIIa, wobei der Rest R¹ die oben genannte Bedeutung hat,
mit oder ohne Lösemittel erhalten werden.

Die Reaktion wird im folgenden Schema am Beispiel 2-(3-Hydroxy-4-methoxyphenyl)-*N*-heptyl-2-hydroxyessigsäureamids verdeutlicht:

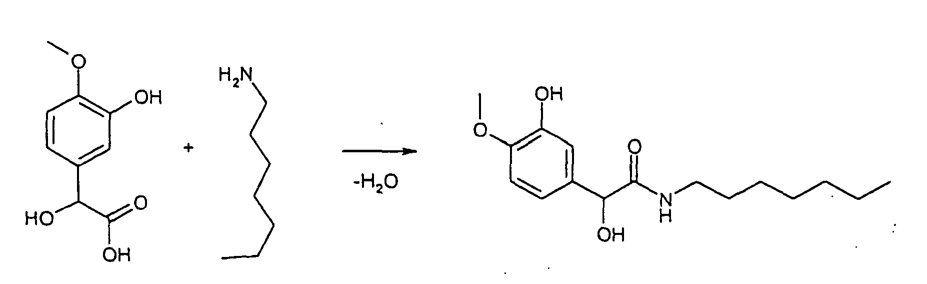

Als Kondensationsmittel können z.B. Carbodiimide, bevorzugt *N,N*'-Dicyclohexylcarbodiimid oder N,N'-Diisopropylcarbodiimid, N,N'-Carbonyldiimidazol, und als Lösemittel bevorzugt aprotische Lösungsmittel, z.B. 1,4-Dioxan, Diethylether, tert.-Butylmethylether, Essigsäureethylester oder Tetrahydrofuran verwendet werden.

Die erfindungsgemäßen Mandelsäurealkylamide erhält man aus diesen Reaktionsgemischen durch an sich bekannte Reinigungsschritte. Vorteilhaft ist das Behandeln einer Lösung der erfindungsgemäßen Mandelsäurealkylamide in einem nicht Wassermischbaren Lösungsmittel, z.B: Essigsäureethylester, Chloroform, Methylenchlorid, aliphatische oder aromatische Kohlenwasserstoffen, Diethylether oder tert.-Butylmethylether, mit einer Mineralsäure, z.B. verdünnter oder konzentrierter Salzsäure, Schwefelsäure, Phosphorsäure, oder einem sauren anorganische oder organischen Ionentauschers, um Reste des eingesetzten Amins der allgemeinen Formel IIIa oder des Ammoniumsalzes IIIb zu entfernen.

Gegebenenfalls müssen noch vorhandene Schutzgruppen mit an sich bekannten Methoden abgespalten werden.

In einer besonders bevorzugten Ausführungsform der Erfindung werden die erfindungsgemäßen Mandelsäurealkylamide in Kombination mit anderen scharf schmeckenden und/oder wärmeerzeugenden Substanzen oder auch scharf schmeckenden pflanzlichen Extrakten verwendet. Auf diese Weise kann ein besonders abgerundetes sensorisches Profil erreicht werden. Insbesondere die Kombination eines oder mehrerer der erfindungsgemäßen Mandelsäureamide mit einem scharf schmeckenden pflanzlichen Extrakt im Verhältnis 0,01 zu 1 bis 100 zu 1, bevorzugt 0,1 zu 1 bis 10 zu 1 erzeugt ein angenehmes sensorisches Profil.

Andere scharf schmeckende und/oder wärmeerzeugende Substanzen können z.B. sein Capsaicin, Dihydrocapsaicin, Gingerol, Paradole, Shogaole, Piperin, Carbonsäure-N-vanillylamide, insbesondere Nonansäure-N-vanillylamid, 2-Alkensäureamide, insbesondere 2-Nonensäure-N-isobutylamid, cis- oder trans-Pellitorin oder Spilanthol, 2-Nonensäure-N-4-hydroxy-3-methoxyphenylamid, Alkylether von 4-Hydroxy-3-methoxybenzylalkohol, insbesondere 4-Hydroxy-3-methoxybenzyl-n-butylether, Alkylether von 4-Acyloxy-3-methoxybenzylalkohol, insbesondere 4-Acetyloxy-3-methoxybenzyl-n-butylether und 4-Acetyloxy-3-methoxybenzyl-n-hexylether, Alkylether von 3-Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, (4-Hydroxy-3-methoxyphenyl)essigsäureamide, insbesonders (4-Hydroxy-3-methoxyphenyl)essigsäure-N-n-octylamid, Ferulasäure-phenethylamiden, Nicotinaldehyd, Methylnicotinat, Propylnicotinat, 2-Butoxyethylnicotinat, Benzylnicotinat, 1-Acetoxychavicol, Polygodial oder Isodrimeninol.

Scharf schmeckende pflanzliche Extrakte können alle für die Ernährung geeigneten pflanzlichen Extrakte sein, die einen scharfen oder warmen sensorischen Eindruck hervorrufen. Bevorzugt als scharf schmeckende pflanzliche Extrakte sind beispielsweise Pfefferextrakt *(Piper ssp.,* insbesondere *Piper nigrum),* Wasserpfefferextrakt (*Polygonum ssp.,* insbesondere *Polygonum hydropiper),* Extrakte aus *Allium ssp.* (insbesondere Zwiebel und Knoblauchextrakte), Extrakte aus Rettich *(Raphanus ssp.),* Meerrettichextrakte *(Cochlearia armoracia),* Extrakte aus schwarzem *(Brassica nigra),* wildem oder gelbem Senf *(Sinapis ssp.,* insbesondere *Sinapis arvensis* und *Sinapis alba),* Bertramwurzel-Extrakte *(Ancyclus ssp.,* insbesondere *Anacylcus pyrethrum* L.), Sonnenhutextrakte *(Echinaceae ssp.),* Extrakten aus Szechuan-Pfeffer *(Zanthoxylum ssp.,* insbeosndere *Zanthoxylum piperitum),* Spilanthesextrakt *(Spilanthes ssp.,* insbesondere *Spilanthes acmella),* Chiliextrakt *(Capsicum ssp.,* insbesondere *Capsicum frutescens),* Paradieskömer-Extrakt *(Aframomum ssp.,* insbesondere *Aframomum melegueta* [Rose] K. Schum.), Ingwerextrakt *(Zingiber ssp.,* insbesondere *Zingiber officinale)* und Galangaextrakt *(Kaempferia galanga* oder *Alpinia galanga).*

Die scharf schmeckenden pflanzlichen Extrakte können aus den entsprechenden frischen oder getrockneten Pflanzen oder Pflanzenteilen, insbesondere aber aus weißen, grünen oder schwarzen Pfefferkörnern, Wasserpfefferkörnern, Zwiebeln und Knoblauch, Rettichwurzel, Meerrettich, Senfkörnern, Sonnenhutwurzeln, Bertramwurzel, Pflanzenteilen der *Zanthoxylum-*Arten, Pflanzenteilen der Spilanthes-Arten, Chilischoten, Paradieskörnern oder Ingwer- oder Galangawurzeln gewonnen werden, dergestalt, dass man die getrockneten Pflanzenteile, die vorzugsweise vorher zerkleinert wurden, mit einem für Nahrungs- und Genussmittel geeigneten Lösungsmittel, vorzugsweise aber Ethanol, Wasser, Hexan oder Heptan oder Ethanol/Wasser-Gemischen, bei 0°C bis zum Siedepunkt des jeweiligen Lösungsmittels oder Gemisches extrahiert, anschließend filtriert und das Filtrat ganz oder teilweise eingeengt, vorzugsweise durch Destillation, Gefrier- oder Sprühtrocknung. Der so erhaltene Rohextrakt kann dann noch weiter aufgearbeitet werden, beispielsweise mit Wasserdampf bei Drücken von 0,01 mbar bis Normaldruck behandelt und/oder in einem für Nahrungs- und Genussmittel geeigneten Lösungsmittel aufgenommen werden.

Ein für Nahrungs- und Genussmittel geeignetes Lösungsmittel kann beispielsweise sein: Wasser, Ethanol, Methanol, Propylenglycol, Glycerin, Aceton, Dichlormethan, Diethylether, Hexan, Heptan oder superkritisches Kohlendioxid oder Gemische der vorgenannten Lösungsmittel.

Weiterer Gegenstand der Erfindung sind der Ernährung oder dem Genuss dienende Zubereitungen, enthaltend die erfindungsgemäßen Mandelsäurealkylamide in einer wirksamen Menge und gegebenenfalls andere übliche Grund-, Hilfs- und Zusatzstoffe für Nahrungs- und Genussmittel. Sie enthalten in der Regel 0,000001 Gew.-% bis 10 Gew.-%, bevorzugt 0,0001 bis 1 Gew.-%, insbesondere aber 0,0001 Gew.-% bis 0,1 Gew.-%, bezogen auf das Gesamtgewicht der Zubereitung, an den Mandelsäurealkylamiden sowie Gemischen derselben. Weitere übliche Grund-, Hilfs- und Zusatzstoffe für Nahrungs- oder Genussmittel können in Mengen von 0,000001 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Die der Ernährung oder dem Genuss dienenden Zubereitungen im Sinne der Erfindung sind z.B. Backwaren (z.B. Brot, Trockenkekse, Kuchen, sonstiges Gebäck), Süßwaren (z.B. Schokoladen, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke, FleischProdukte (z.B. Schinken, Frischwurst- oder Rohwurstzubereitungen), Eier oder Eiprodukte (Trockenei, Eiweiß, Eigelb), Getreideprodukte (z.B. Frühstückscerealien, Müsliriegel), Milchprodukte (z.B. Milchgetränke, Milcheis, Joghurt, Kefir, Frischkäse, Weichkäse, Hartkäse, Trockenmilchpulver, Molke, Butter, Buttermilch), Fruchtzubereitungen (z.B. Konfitüren, Fruchteis, Fruchtsoßen), Gemüsezubereitungen (z.B. Ketchup, Soßen, Trockengemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings), Fertiggerichte und Suppen, Gewürze, Würzmischungen sowie insbesondere Aufstreuwürzungen (Seasonings), die im Snackbereich Anwendung finden. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Besonders vorteilhaft hat sich auch erwiesen, dass die erfindungsgemäßen Mandelsäurealkylamide, insbesondere die erfindungsgemäßen Mandelsäurealkylamide in Kombination mit scharf schmeckenden pflanzlichen Extrakten, den scharfen Geschmack von Alkohol in alkoholischen Getränken oder Zubereitungen aus alkoholischen Getränken imitieren können und es damit möglich ist, den Alkoholgehalt in alkoholischen Getränken oder in Zubereitungen aus alkoholischen Getränken bei gleichbleibender sensorischer Beurteilung niedriger einzustellen.

Besonders vorteilhaft hat sich auch erwiesen, dass die erfindungsgemäßen Mandelsäurealkylamide, den scharfen Geschmack von Capsaicin, Dihydrocapsaicin und Nonivamid imitieren können und es damit möglich ist, den Capsaicingehalt in den der Ernährung, der Mundhygiene oder dem Genuss dienenden Zubereitungen bei gleichbleibender sensorischer Beurteilung niedriger einzustellen.

Ein weitere bevorzugte Ausführung der Erfindung sind der Mundhygiene dienende Zubereitungen, insbesondere Zahnpflegemittel wie Zahnpasten, Zahngele, Zahnpulver, Mundwässer, Kaugummis und andere Mundpflegemittel, enthaltend die Mandelsäurealkylamide in einer wirksamen Menge und gegebenenfalls andere übliche Grund-, Hilfs- und Zusatzstoffe für solche Zubereitungen. Sie enthalten in der Regel 0,000001 Gew.-% bis 10 Gew.-%, bevorzugt 0,0001 bis 1 Gew.-%, insbesondere aber 0,0001 Gew.-% bis 0,1 Gew.-%, bezogen auf das Gesamtgewicht der Zubereitung, an den Mandelsäurealkylamiden sowie Gemischen derselben. Weitere übliche Grund-, Hilfs- und Zusatzstoffe für die der Mundhygiene dienenden Zubereitungen können in Mengen von 0,000001 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Zahnpflegemittel, enthalten die erfindungsgemäßen Mandelsäurealkylamide, bestehen im allgemeinen aus einem abrasiven System (Schleif- oder Poliermittel), wie z.B. Kieselsäuren, Calciumcarbonaten, Calciumphosphaten, Alumiuniumoxiden und/oder Hydroxylapatiten, aus oberflächenaktiven Substanzen, wie z.B. Natriumlaurylsulfat, Natriumlaurylsarcosinat und/oder Cocamidopropylbetain, aus Feuchthaltemitteln, wie z.B. Glycerin und/oder Sorbit, aus Verdickungsmitteln, wie z.B. Carboxymethylcellulose, Polyethylenglycolen, Carrageenanen und/oder Laponiten® , aus Süßstoffen, wie z.B. Saccharin, aus Stabilisatoren und aus aktiven Wirkstoffen, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat.

Kaugummis, enthalten die erfindungsgemäßen Mandelsäurealkylamide, bestehen im allgemeinen aus einer Kaugummibase, d.h. einer beim Kauen plastische werdenden Kaumasse, aus Zuckern verschiedener Arten, Zuckeraustauschstoffen, Süßstoffen, Zuckeralkoholen, Feuchthaltemitteln, Verdickern, Emulgatoren, Stabilisatoren und Aromen.

Die erfindungsgemäßen Zubereitungen, enthaltend die erfindungsgemäßen Mandelsäurealkylamide können dergestalt hergestellt werden, dass die erfindungsgemäßen Mandelsäurealkylamide als Substanz, als Lösung oder in Form eines Gemisches mit einem festen oder flüssigen Trägerstoff in die der Ernährung, der Mundhygiene oder dem Genuss dienenden Zubereitungen eingearbeitet werden.

Zur Herstellung der Zubereitungen können in einer weiteren bevorzugten Ausführungsform die erfindungsgemäßen Mandelsäurealkylamide und gegebenen-falls andere Bestandteile der erfindungsgemäßen Zubereitung auch vorher in Emulsionen, in Liposomen, z.B. ausgehend von Phosphatidylcholin, in Microsphären, in Nanosphären oder auch in Kapseln aus einer für Lebens- und Genussmittel geeigneten Matrix, z.B. aus Stärke, Stärkederivaten, anderen Polysacchariden, natürlichen Fetten, natürlichen Wachsen oder aus Proteinen, z.B. Gelatine, eingearbeitet werden. Eine weitere Ausführungsform besteht darin, dass die erfindungsgemäßen Mandelsäurealkylamide vorher mit geeigneten Komplexbildnern, beispielsweise mit Cyclodextrinen oder Cyclodextrinderivaten, bevorzugt ß-Cyclodextrin, komplexiert werden und in dieser Form eingesetzt werden.

Als andere Bestandteile für die erfindungsgemäßen, der Ernährung oder dem Genuss dienenden Zubereitungen können weitere übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Ei, Knochen, Knorpel, Fisch, Krusten- und Schalentiere, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit, Mannitol, Xylitol), natürliche oder gehärtete Fette (z.B. Talg, Schmalz, Palmfett, Kokosfett, gehärtetes Pflanzenfett), fette Öle (z.B. Sonnenblumenöl, Erdnussöl, Maiskeimöl, Distelöl, Olivenöl, Walnussöl, Fischöl, Sojaöl, Sesamöl), Fettsäuren oder deren Salze (z.B. Kaliumstearat, Kaliumpalmitat), proteinogene oder nicht-proteinogene Aminosäuren und verwandte Verbindungen (z.B. Taurin, Kreatin, Kreatinin), Peptide, native oder prozessierte Proteine (z.B. Gelatine), Enzyme (z.B. Peptidasen, Glucosidasen, Lipasen), Nukleinsäuren, Nucleotide (Inositolphosphat), geschmacksmodulierende Stoffe (z.B. Natriumglutamat, 2-Phenoxypropionsäure), Emulgatoren (z.B. Lecithine, Diacylglycerole), Stabilisatoren (z.B. Carageenan, Alginat, Johannisbrotkernmehl, Guarkernmehl), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin), Süßstoffe (z.B. Saccharin, Cyclamat, Aspartam, Neotam, Neohesperidindi-hydrochalkon), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, sowie Riechstoffe, synthetische, natürliche oder naturidentische Aroma- und Geschmackstoffe.

Bevorzugt können die erfindungsgemäßen Zubereitungen auch noch eine Aromakomposition enthalten, um den Geschmack und/oder Geruch der Zubereitung abzurunden und zu verfeinern. Geeignete Aromakompositionen enthalten z.B. synthetische, natürliche oder naturidentische Aromastoffe sowie Riechstoffe, insbesondere aber auch andere scharf schmeckende und/oder wärmeerzeugende Substanzen oder Pflanzenextrakte.

Ein weiterer Gegenstand der Erfindung ist die Verwendung der erfindungsgemäßen Zubereitungen als Halbfertigwaren zur Aromatisierung von daraus gefertigten Zubereitungen als Fertigwaren.

### Beispiele

### Darstellung der Amide

Die Mandelsäure (2,53 mmol) und *N*-Hydroxysuccinimid (2,53 mmol) werden in trocknem 1,4-Dioxan (20 ml) unter Stickstoff vorgelegt und bei Raumtemperatur wird *N*-*N*'-Dicyclohexylcarbodiimid (2,53 mmol) zugegeben. Die trübe werdende Mischung wird bei Raumtemperatur 16 h gerührt und über eine Glasfritte (P3) filtriert. Das Filtrat wird zu einer Lösung des Amins oder Ammoniumhydrochlorids (3,03 mmol) in Wasser (10 ml) gegeben und NaHCO₃ (3,03 mmol) hinzugefügt. Die resultierende Mischung wird bei 50 °C 1,5 h gerührt, dann mit 5 %-Salzsäure auf pH < 2 gebracht, mit Essigsäureethylester 3-mal extrahiert, die vereinigten organischen Phasen mit ges. NaCl-Lsg. und gegebenenfalls nochmals mit Salzsäure gewaschen, über Na₂SO₄ getrocknet, filtriert und bei 40°C/230-20 mbar eingedampft. Der Rückstand wird ggfs. an Kieselgel 60 chromatographiert und/oder umkristallisiert.

### Beispiel 1: 2-(4-Hydroxyphenyl)-2-hydroxy-N-heptylessigsäureamid(nicht erfindungsgemäß):

Ausbeute: 28 % nach Rekristallisation; Reinheit > 98 % (HPLC); HPLC-MS (APCI+) m/z = 266,11 (100 %, [M+H]⁺), 249,35 (84,6 %, [M-HO+H]⁺), 530,61 (22,6 %, [2M+H]⁺); ¹H-NMR (200 MHz; CD₃OD) δ = 7,23 (2H, m, AA'), 6,74 (2H, m, BB'), ca. 4,9 ppm (s, unter D₂O-Signal), 3.21 (2H, t, 7 Hz), 1,50 (2H, m), 1,40-1,20 (8H, m), 0,88 (3H, t, 7 Hz) ppm; ¹³C-NMR (50 Hz, CD₃OD) 175,68 (C), 158,36 (C), 132,55 (C), 129,15 (2 x CH), 115,95 (2 x CH), 75,12 (CH), 40,10 (CH₂), 39,98 (CH₂), 32,91 (CH₂), 30,49 (CH₂), 30,05 (CH₂), 27,82 (CH₂), 23,61 (CH₂), 14,43 (CH₃) ppm.

### Beispiel 2: 2-(4-Hydroxyphenyl)-2-hydroxy-N-octylessigsäureamid (nicht erfindungsgemäß);

Ausbeute: 52 % nach Rekristallisation; Reinheit > 95 % (HPLC); HPLC-MS (APCI+) *m*/*z* = 280,07 (100 %, [M+H]⁺), 262,61 (84, %, [M-H₂O+H]⁺), 558,68 (15,3 %, [2M+H]⁺); ¹H-NMR (200 MHz; CD₃OD) δ = 7,23 (2H, m, AA'), 6,74 (2H, m, BB'), 4,89 ppm (s, unter D₂O-Signal) 3.21 (2H, t, 7 Hz), 1,50 (2H, m), 1,40-1,20 (10H, m), 0,89 (3H, t, 7 Hz) ppm; ¹³C-NMR (50 Hz, CD₃OD) 175,60 (C), 158,36 (C), 132,55 (C), 129,15 (2 x CH), 115,95 (2 x CH), 75,11 (CH), 40,10 (CH₂), 39,98 (CH₂), 32,91 (CH₂), 30,48 (CH₂), 30,34 (2 x CH₂), 27,87 (CH₂), 23,68 (CH₂), 14,44 (CH₃) ppm.

### Beispiel 3: 2-(4-Hydroxyphenyl)-2-hydroxy-N-nonylessigsäureamid(nicht erfindungs- gemäβ

Ausbeute: 76 % nach Rekristallisation; Reinheit > 90 % (HPLC); HPLC-MS . (APCI+) *mlz* = 294,10 (100 %, [M+H]⁺), 276,51 (61,8 %, [M-H₂O+H]⁺), 586,75 (6,45 %, [2M+H]⁺); ¹H-NMR (200 MHz; CD₃OD) δ= 7,22 (2H, m, AA'), 6,71 (2H, m, BB'), 4,89 ppm (s, unter D₂O-Signal), 3.21. (2H, t, 7 Hz), 1,50 (2H, m), 1,40-1,20 (12H, m), 0,88 (3H, t, 7 Hz) ppm; ¹³C-NMR (50 Hz, CD₃OD) 175,59 (C), 158,36 (C), 132,54 (C), 129,15 (2 x CH), 115,95 (2 x CH), 75,09 (CH), 39,98 (CH₂), 32,99 (CH₂), 30,63 (CH₂), 30,47 (CH₂), 30,38 (CH₂), 30,34 (CH₂), 27,87 (CH₂), 23,69 (CH₂), 14,45 (CH₃) ppm.

### Beispiel 4: 2-(3,4-Dihydroxyphenyl)-2-hydroxy-N-octylessigsäureamid(nicht erfindungsgemäß):

Ausbeute: 16 % nach Chromatographie; HRMS(Direkteinlass): gemessen 295,17650, berechnet 295,17834 für C₁₆H₂₅NO₄; HPLC-MS (ESI+) m/z = 277,95 (100 %, [M-H₂O+H]⁺), 295,80 (92,76 %, [M+H]⁺), 590,63 (23,3 %, [2M+H]⁺); ¹H-NMR (200 MHz; CD₃OD) δ = 6,84 (1H, m), 6,73-6,71 (2H, m), 4,84 ppm (1H, s), 3,21 (2H, t, 7 Hz), 1,50 (2H, m), 1,40-1,20 (10H, m), 0,89 (3H, t, 7 Hz) ppm; ¹³C-NMR (50 Hz, CD₃OD) 175,57 (C), 146,25 (C), 146,09 (C), 133,14 (C), 119,61 (CH), 115,93 (CH), 115,08 (CH), 75,22 (CH), 40,03 (CH₂), 32,92 (CH₂), 30,49 (CH₂), 30,35 (2 x CH₂), 27,90 (CH₂), 23,68 (CH₂), 14,45 (CH₃) ppm.

### Beispiel 5: 2-(3-Hydroxy-4-methoxyphenyl)-2-hydroxy-N-heptylessigsäureamid:

Ausbeute: 47 % nach Chromatographie; Reinheit > 95 % (HPLC); HPLC-MS (APCI+) *m*/*z* = 278,51 (100 %, [M+H-H₂O]⁺), 295,97 (84,6 %, [M+H]⁺), 590,81 (20,4 %, [2M+H]⁺); HRMS(Direkteinlass): gemessen 295,17699, berechnet 295,17834 für C₁₆H₂₅NO₄; ¹H-NMR (400 MHz; CD₃OD; mit Wasserunterdrückung) δ = 6,89 (1H, s), 6,86 (2H, m), ca. 4,9 ppm (unterdrückt), 3,83 (3H, s), 3,22 (2H, td, 7 Hz, 1 Hz), 1,51 (2H, tt, 7 Hz, 7 Hz), 1,35-1,22 (8H, m), 0,90 (3H, t, 7 Hz) ppm; ¹³C-NMR (100 Hz, CD₃OD) 175,67 (C), 149,03 (C), 147,59 (C), 134,75 (C), 119,56 (CH), 114,97 (CH), 112,41 (CH), 75,25 (CH), 56,43 (CH₃), 40,08 (CH₂), 32,99 (CH₂), 30,57 (CH₂), 30,14 (CH₂), 27,90 (CH₂), 23,67 (CH₂), 14,46 (CH₃) ppm.

### Beispiel 6: 2-(3-Hydroxy-4-methoxyphenyl)-2-hydroxy-N-octylessigsäureamid:

Ausbeute: 36 % nach Chromatographie; Reinheit > 95 % (HPLC); HPLC-MS (APCI+) m/z = 292,66 (100 %, [M+H H₂O]⁺), 310,01 (69,6 %, [M+H]⁺), 618,83 (46 %, [2M]); HRMS(Direkteinlass): gemessen 309,19519, berechnet 309,19400 für C₁₇H₂₇NO₄; ¹H-NMR (400 MHz; CD₃OD; mit Wasserunterdrückung) δ = 6,88 (1H, m), 6,85 (2H, m), ca. 4,9 ppm (unterdrückt), 3,83 (3H, s), 3,21 (2H, td, 7 Hz, 1 Hz), 1,50 (2H, m), 1,40-1,22 (10H, m), 0,88 (3H, t, 7 Hz) ppm;

### Beispiel 7: 2-(3-Hydroxy-4-methoxyphenyl)-2-hydroxy-N-nonylessigsäureamid:

Ausbeute: 50 %, farblose Kristalle; Reinheit > 95 % (HPLC); HPLC-MS (APCI+) m/z = 324,16 (100 %, [M+H]⁺), 306,60 (80 %, [M+H- H₂O]⁺), 646,88 (47 %, [2M+H]⁺); HRMS(Direkteinlass): gemessen 323,20822, berechnet 323,20966 für C₁₈H₂₉NO₄; ¹H-NMR (200 MHz; CD₃OD) δ = 6,88 (1H, m), 6,86 (2H, m), 4,87 ppm (1H, s), 3,82 (3H, s), 3,21 (2H, t, 7 Hz), 1,51 (2H, m), 1,40-1,25 (12H, m), 0,88 (3H, t, 7 Hz) ppm.

### Beispiel 8: 2-(4-Hydroxy-3-methoxyphenyl)-2-hydroxy-N-heptylessigsäureamid:

Ausbeute: quantitativ; Reinheit > 95 % (HPLC); HPLC-MS (APCI+) *m*/*z* = 278,61 (100 %, [M+H- H₂O]⁺), 296,17 (27 %, [M+H]⁺), 590,78 (12 %, [2M+H]⁺); HRMS(Direkteinlass): gemessen 295,17679, berechnet 295,17834 für C₁₆H₂₅NO₄; ¹H-NMR (200 MHz; CD₃OD; mit Wasserunterdrückung) δ= 7,00 (1H, d, 2 Hz), 6,86 (1H, dd, 8 Hz, 2 Hz), 6,74 (1H, d, 8 Hz), ca. 4,9 ppm (unterdrückt), 3,83 (3H, s), 3,21 (2H, td, 7 Hz, 1 Hz), 1,50 (2H, m), 1,40-1,20 (12H, m), 0,89 (3H, t, 7 Hz) ppm; ¹³C-NMR (50 Hz, CD30D) 175,60 (C), 148,74 (C), 147,49 (C), 133,17 (C), 120,76 (CH), 115,82 (CH), 111,34 (CH), 75,36 (CH), 56,27 (CH₃), 40,01 (CH₂), 32,96 (CH₂), 30,55 (CH₂), 30,11 (CH₂), 27,87 (CH₂), 23,65 (CH₂), 14,44 (CH₃) ppm.

### Beispiel 9: 2-(4-Hydroxy-3-methoxyphenyl)-2-hydroxy-N-octylessigsäureamid:

Ausbeute: 76 % nach Chromatographie; Reinheit > 95 % (HPLC); HPLC-MS (APCI+) *m*/*z* = 292,40 (100 %, [M+H- H₂O]⁺), 309,90 (31 %, [M+H]⁺), 618,71 (5 %, [2M+H]⁺); HRMS(Direkteinlass): gemessen 309,19209, berechnet 309,19400 für C₁₇H₂₇NO₄; ¹H-NMR (200 MHz; CD₃OD) δ = 7,01 (1H, d, 2 Hz), 6,88 (1H, dd, 8 Hz, 2 Hz), 6,76 (1H, d, 8 Hz), ca. 4,9 ppm (unter dem HDO-Signal), 3,85 (3H, s), 3,21 (2H, td, 7 Hz, 1 Hz), 1,51 (2H, m), 1,38-1,22 (10H, m), 0,89 (3H, t, 7 Hz) ppm; ¹³C-NMR (50 Hz, CD₃OD) 175,60 (C), 148,76 (C), 147,50 (C), 133,19 (C), 120,78 (CH), 115,83 (CH), 111,37 (CH), 75,36 (CH), 56,29 (CH₃), 40,02 (CH₂), 32,96 (CH₂), 30,56 (CH₂), 30,41 (2 □ CH₂), 27,92 (CH₂), 23,73 (CH₂), 14,45 (CH₃) ppm.

### Beispiel 10: 2-(4-Hydroxy-3-methoxyphenyl)-2-hydroxy-N-nonylessigsäureamid:

Ausbeute: 1,5 g nach Chromatographie; Reinheit > 95 % (HPLC); HPLC-MS (APCI+) m/z = 306,43 (100 %, [M+H- H₂O]⁺), 323,21 (22,5 %), 324,08 (21,8 %, [M+H]⁺); ERMS(Direkteinlass): gemessen 323,20855, berechnet 323,20966 für C₁₈H₂₉NO₄; ¹H-NMR (200 MHz; CD₃OD; mit Wasserunterdrückung) δ = 7,01 (1H, d, 2 Hz), 6,88 (1H, dd, 8 Hz, 2 Hz), 6,74 (1H, d, 8 Hz), ca. 4,9 ppm (unterdrückt), 3,85 (3H, s), 3,22 (2H, td, 7 Hz, 1 Hz), 1,60-1,40 (2H, m), 1,40-1,20 (12H, m), 0,89 (3H, t, 7 Hz) ppm; ¹³C-NMR (50 Hz, CD₃OD) 175,57 (C), 148,72 (C), 147,47 (C), 133,14 (C), 120,75 (CH), 115,81 (CH), 111,33 (CH), 75,33 (CH), 56,26 (CH₃), 39,99 (CH₂), 33,04 (CH₂), 30,68 (CH₂), 30,53 (CH₂), 30,43 (CH₂), 30,38 (CH₂), 27,91 (CH₂), 23,72 (CH₂), 14,47 (CH₃) ppm.

### Beispiel 11: Verkostung der Mandelsäurealkylamide

Die zu verkostende Substanz wird in Ethanol gelöst und die ethanolische Lösung dann mit 11 %iger Zuckerlösung verdünnt (Endkonzentration: c). Zur Verkostung werden jeweils ca. 5 ml der Zuckerlösung heruntergeschluckt. Wenn der Schwellenwert der Substanz bekannt ist, wird für die Verkostung ein Wert knapp über dem Schwellenwert gewählt. Eine Gruppe von 6 - 8 Prüfern hat die Lösungen verkostet.

Der Schärfeeindruck wurde wenn möglich auf einer Skala 1 (sehr schwach) - 9 (sehr stark) eingeschätzt.
a) Profil 2-(3,4-Dihydroxyphenyl)-2-hydroxy-N-octylessigsäureamid (Nr. 4) (nicht erfindungsgemäß, 10 ppm: Schärfe entwickelt sich langsam; stechend, kratzend, leicht würzig; Einschätzung der Schärfe 5.
b) Profil 2-(3-Hydroxy-4-methoxyphenyl)-2-hydroxy-N-octylessigsäureamid (Nr. 6): 1 ppm: leicht scharf, vor Allem auf der Zunge, leichtes Tingling, verschwindet schnell wieder; Einschätzung der Schärfe 3.
c) Profil 2-(4-Hydroxy-3-methoxyphenyl)-2-hydroxy-N-heptylessigsäureamid (Nr. 8):
   10 ppm: Schärfe entwickelt sich; brennend, leicht überreif; Einschätzung der Schärfe 7.
d) Profil 2-(4-Hydroxy-3-methoxyphenyl)-2-hydroxy-N-octylessigsäureamid (Nr. 9): 1 ppm: erst gar keine Schärfe, entwickelt sich schlagartig, sehr intensiv und langanhaltend, Ingwerschärfe; Einschätzung der Schärfe 8.
e) Profil 2-(4-Hydroxy-3-methoxyphenyl)-2-hydroxy-N-nonylessigsäureamid (Nr. 10):
   10 ppm: Schärfe setzt sofort ein; ähnlich zu Capsaicin; Einschätzung der Schärfe 8.

### Vergleichsbeispiele

### f) Profil Dihydrocapsaicin:

100 ppb: leicht verzögert einsetzende Wirkung im Rachenraum, brennend, aggressiv, keine Wärmeentwicklung.

### g) Profil N-(3-Methoxy-4-hydroyxbenzyl)nonansäureamid

200 ppb: leicht verzögert einsetzende Wirkung im Rachenraum, wenig Schärfe auf der Zunge, stechend, keine Wärmeentwicklung

### Beispiel 12: Anwendung in einer Zahnpasta als Aromastoff

| **Teil** | **Inhaltsstoff** | **Einsatz in Gew.-%** |
|---|---|---|
| A | demineralisiertes Wasser | 22,00 |
| | Sorbitol (70%) | 45,00 |
| | Solbrol® M, Natriumsalz (Bayer AG, p-Hydroxybenzoesäurealkylester) | 0,15 |
| | Trinatriumphosphat | 0,10 |
| | Saccharin, 450 fach | 0,20 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisertes Wasser | 4,53 |
| | Natriumlaurylsulfat | 1,50 |
| D | Aroma, enthaltend 0,1 % 2-(4-Hydroxy-3-methoxyphenyl)-2-hydroxy-N-octylessigsäureamid | 1 |

Die Inhaltsstoffe der Teile A und B werden jeweils für sich vorgemischt und zusammen unter Vakuum bei 25 - 30°C 30 min gut verrührt. Teil C wird vorgemischt und zu A und B gegeben; D wird hinzugefügt und die Mischung unter Vakuum bei 25 - 30°C 30 min gut verrührt. Nach Entspannung ist die Zahnpasta fertig und kann abgefüllt werden.

### Beispiel 13: Anwendung in einem zuckerfreien Kaugummi als Aromastoff

| **Teil** | **Inhaltsstoff** | **Einsatz in Gew.-%** |
|---|---|---|
| A | Kaugummibase, Company "Jagum T" | 30,00 |
| B | Sorbit, pulverisiert | 39,00 |
| | Isomalt® (Palatinit GmbH) | 9,50 |
| | Xylit | 2,00 |
| | Mannit | 3,00 |
| | Aspartam® | 0,10 |
| | Acesulfam® K | 0,10 |
| | Emulgum® (Colloides Naturels, Inc.) | 0,30 |
| C | Sorbitol, 70% | 14,00 |
| | Glycerin | 1,00 |
| D | Aroma, enthaltend 0,1 % 2-(4-Hydroxy-3-methoxyphenyl)-2-hydroxy-N-heptylessigsäureamid | 1 |

Teile A bis D werden gemischt und intensiv geknetet. Die Rohmasse kann z.B. in Form von dünnen Streifen zu verzehrsfertigen Kaugummis verarbeitet werden.

### Beispiel 14: Anwendung in einem Mundwasser als Aromastoff

| **Teil** | **Inhaltsstoff** | **Gehalt (%)** |
|---|---|---|
| A | Ethanol | 10,00 |
| | Cremophor® CO 40 (BASF, Detergenz) | 1,00 |
| | Benzoesäure | 0,12 |
| | Aroma, enthaltend 0,4 % 2-(4-Hydroxy-3-methoxyphenyl)-2-hydroxy-N-nonylessigsäureamid | 0,25 |
| B | demineralisiertes Wasser | 83,46 |
| | Sorbitol, 70% | 5,00 |
| | Natriumsaccharin 450 | 0,07 |
| | L-Blue 5000 e.c., 1% in Wasser (Farbstoff) | 0,10 |

Die Inhaltsstoffe der Teile A und B werden jeweils für sich gemischt. Teil B wird langsam in Teil A eingerührt, bis die Mischung homogen ist.

### Beispiel 15: Anwendung in Kombination mit einem scharfen Pflanzenextrakt als Alkoholverstärker

**Vergleichsprobe Likörbase 10%vol**

| | |
|---|---|
| 7,39 kg | Alkohol, p.A.-Ware |
| 20 kg | Invertzuckersirup, 66,5 % Trockenmasse |
| 72,61 kg | Wasser |
| Summe 100 kg | |

**Likörbase 5,5%vol**

| | |
|---|---|
| 4,06 kg | Alkohol, p.A.-Ware |
| 20 kg | Invertzuckersirup, 66,5 % Trockenmasse |
| 75,94 kg | Wasser |
| Summe 100 kg | |

Version A: Likörbase 5,5 %vol + 0,3 % einer 10 %igen Lösung eines Paradieskörnerextrakts in Ethanol

Version B: Likörbase 5,5 %vol + 0,075% einer 10 %igen Lösung eines Paradieskömerextrakts in Ethanol + 0,02% einer Lösung von 2-(4-Hydroxy-3-methoxyphenyl)-2-hydroxy-N-nonylessigsäureamid 1%ig in Ethanol (entspricht 2 ppm).

Bei Version B wird sensorisch die Alkoholschärfe der Vergleichsprobe besser imitiert als in Version A. Version A und die Vergleichsprobe sind sensorisch sehr ähnlich zu bewerten.

## Patentansprüche

1. Mandelsäurealkylamide der allgemeinen Formel (I) 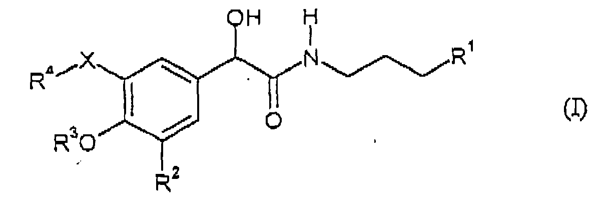 wobei
R¹ einen linearen oder verzweigten Alkylrest mit 1 bis 20 Kohlenstoffatomen oder einen linearen oder verzweigten Alkenylrest mit 2 bis 20 Kohlenstoffatomen darstellt und
R². ein Wasserstoffatom darstellt,
und
entweder
X eine Einfachbindung,
R³ einen Niederalkylrest oder einen Niederakenylrest und
R⁴ Wasserstoff darstellen
oder
X ein Sauerstoffatom,
R³ Wasserstoff und
R⁴ einen Niederalkylrest oder einen Niederalkenylrest darstellt
oder
X ein Sauerstoffatom.
R³ einen Niederalkylrest oder einen Niederalkenylrest und
R⁴ Wasserstoff darstellt
und deren verschiedenen Stereoisomeren oder deren Gemische mit der Ausnahme, dass X ein Sauerstoffatom, R¹ 1-Pentyl, R² und R³ Wasserstoff und R⁴ Methyl darstellen.

2. 2-(4-Methoxyphenyl)-2-hydroxy-N'-heptylessigsäureamid,
2-(4-Methoxyphenyl)-2-hydroxy-N-octylessigsäureamid,
2-(4-Methoxyphenyl)-2-hydroxy-N-nonylessigsäureamid,
2-(3-Hydroxy-4-methoxyphenyl)-2-hydroxy-N-heptylessigsäureamid,
2-(3-Hydroxy-y-methoxyphenyl)-z-hydroxy-N-octylessigsäureamid,
2-(3-Hydroxy-4-methoxyphenyl)-2-hydroxy-N-nonylessigsäureamid,
2-(4-Hydroxy-3-methoxyphenyl)-2-hydroxy-N-heptylessigsäureamid,
2-(4-Hydroxy-3-methoxyphenyl)-2-hydroxy-N-nonylessigsäureamid,
und
2-(4-Hydroxy-3-methoxyphenyl)-2-hydroxy-N-(7-methyl-1-octyl)essigsäureamid.

3. Verfahren zur Herstellung der Verbindungen nach Anspruch 1 oder 2, **dadurch gekennzeichnet, dass** eine Mandelsäure der allgemeinen Formel II 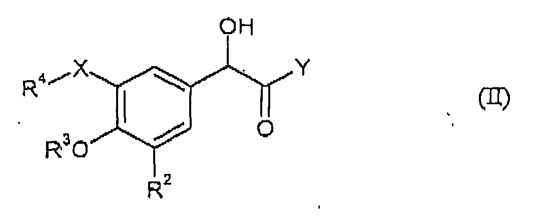 wobei
X, R², R³ und R⁴ die in Anspruch 1 genannte Bedeutung haben,
und
Y eine aktivierte Abgangsgruppe darstellt,
oder Derivaten, deren OH-Gruppen mit Schutzgruppen geschützt sind, mit einem Alkylamin der allgemeinen Formel (IIIa) 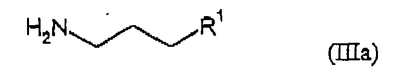 oder einem Alkylammoniumsalz der allgemeinen Formel (IIIb) 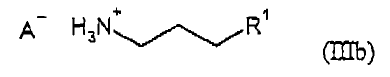 wobei R¹ die oben angeführte Bedeurung hat und A⁻ ein anorganisches oder organisches Anion bedeutet,
gegebenenfalls in Gegenwart von Lösemitteln und/oder Hilfsbasen umgesetzt und gegebenenfalls die Schutzgruppen der OH-Gruppen abgespaltet werden.

4. Der Ernährung, der Mundhygiene oder dem Genuss dienende Zubereitungen, enthaltend Mandelsäuxealkylamide der allgemeinen Formel (I) 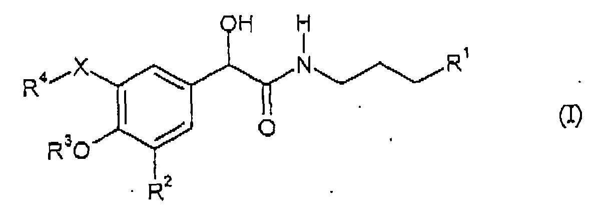 wobei
R¹ einen linearen oder verzweigten Alkylrest mit 1 bis 20 Kohlenstoffatomen oder einen linearen oder verzweigten Alkenylrest mit 2 bis 20 Kohlenstoffatomen darstellt und
R² ein Wasserstoffatom darstellt,
und
entweder
X eine Einfachbindung,
R³ einen Niederadkylrest oder einen Niederalkenylrest und
R⁴ Wasserstoff darstellen
oder
X ein Sauerstoffatom,
R³ Wasserstoff und
R⁴ einen Niederalkylrest oder einen Niederalkenylrest darstellt
oder
X ein Sauerstoffatom,
R³ einen Niederalkylrest oder einen Niederalkenylrest und
R⁴ Wasserstoff darstellt
und deren verschiedenen Stereoisomeren oder deren Gemische

5. Zubereitungen nach Anspruch 4, enthaltend mindestens eine weitere scharf schmeckende oder wärmeerzeugende Substanz.

6. Zubereitungen nach Anspruch 4, enthaltend mindestens einen scharf schmeckenden pflanzlichen Extrakt.

7. Zubereitungen nach Anspruch 4, enthaltend mindestens eine weitere scharf schmeckende oder wärmeerzeugende Substanz und mindestens einen scharf schmeckenden pflanzlichen Extrakt.

8. Als Halbfertigwaren vorliegende Zubereitungen nach mindestens einem der Ansprüche 4 bis 7.

9. Als Riech-, Aroma- und Geschmacksstoffkompositionen sowie Würzmischungen vorhegende Zubereitungen nach mindestens einem der Ansprüche 4 bis 8.

10. Kosmetische oder dermatologische Zubereitungen, enthaltend Mandelsäuzealkylamide der allgemeinen Formal (I) 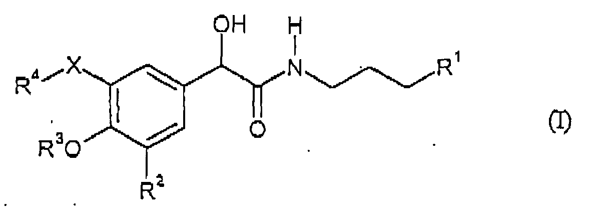 wobei, X, R¹, R², R³ und R⁴ oder in Aupruch 4 angegebene Bedeutung haben,
und deren verschiedenen Stereoisomeren oder deren Gemische

11. Verwendung von Mandelsäurealkylamiden der allgemeinen Formel (I) 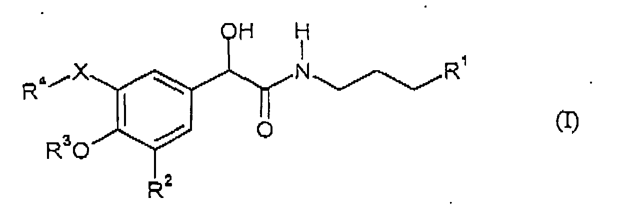 wobei X, R¹, R², R³und R⁴ oder in Auspruch 4 angegebene Bedeutung haben,
und deren Verschiedenen Stereoisomeren oder deren Gemische als Aromastoffe.

12. Verwendung von
2-(4-Methoxyphenyl)-2-hydroxy-N-heptylessigsäureamid,
2-(4-Methoxyphenyl)-2-hydroxy-N-octylessigsäureamid,
2-(4-Methoxyphenyl)-2-hydroxy-N-nonylessigsäureamid,
2-(3-Hydroxy-4-methoxyphenyl)-2-hydroxy-N-heptylessigsäureamid,
2-(3-hydroxy-y-methoxyphenyl)-2-hydroxy-N-octylessigsäureamid,
2-(3-Hydroxy-4-methoxyphenyl)-2-hydroxy-N-nonylessigsäureamid,
2-(4-Hydroxy-3-methoxyphenyl)-2-hydroxy-N-heptylessigsäureamid,
2-(4-Hydroxy-3-methoxyphenyl)-2-hydroxy-N-nonylessigsäureamid,
2-(4-hydroxy-3-methoxyphenyl)-2-hydroxy-N-octylessigsäureamid
und
2-(4-Hydroxy-3-methoxyphenyl)-2-hydroxy-N-(7-methyl-1-octyl)essigsäureamid
und deren verschiedenen Stereoisomeren oder deren Gemische als Aromastoffe.

13. Verwendung nach Anspruch 11 oder 12 wobei Aromastoff Scharfstoff oder Aromastoff mit einem wärmeerzeugenden Effekt bedeutet.

14. Verwendung nach mindestens einem der Ansprüch 11 bis 13 in der Ernährung oder dem Genuss dienenden Zubereitungen.

15. Verwendung nach mindestens einem der Ansprüche 11 bis 14 in der Mundhygiene dienenden Zubereitungen.

16. Verwendung der Mandelsäurealkylamide der Formel (I), wobei X, R¹, R², R³ und R⁴ die in Anspruch 4 angegebene Bedeutung haben, in kosmetischen oder dermatologischen Zubereitungen.

## Claims

1. Mandelic acid alkylamides of the general formula (I) 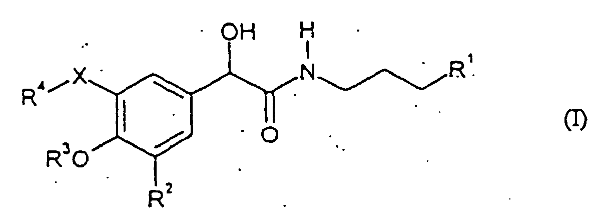 wherein
R¹ represents a linear or branched alkyl radical having from 1 to 20 carbon atoms or a linear or branched alkenyl radical having from 2 to 20 carbon atoms and
R² represents a hydrogen atom,
and
either
X represents a single bond,
R³ represents a lower alkyl radical or a lower alkenyl radical and
R⁴ represents hydrogen,
or
X represents an oxygen atom,
R³ represents hydrogen and
R⁴ represents a lower alkyl radical or a lower alkenyl radical,
or
X represents an oxygen atom,
R³ represents a lower alkyl radical or a lower alkenyl radical and
R⁴ represents hydrogen,
and the various stereoisomers thereof or mixtures thereof, with the exception of compounds wherein X represents an oxygen atom, R¹ represents 1-pentyl, R² and R³ represent hydrogen and R⁴ represents methyl.

2. 2-(4-methoxyphenyl)-2-hydroxy-N-heptylacetic acid amide,
2-(4-methoxyphenyl)-2-hydroxy-N-octylacetic acid amide,
2-(4-methoxyphenyl)-2-hydroxy-N-nonylacetic acid amide,
2-(3-hydroxy-4-methoxyphenyl)-2-hydroxy-N-heptylacetic acid amide,
2-(3-hydroxy-4-methoxyphenyl)-2-hydroxy-N-octylacetic acid amide,
2-(3-hydroxy-4-methoxyphenyl)-2-hydroxy-N-nonylacetic acid amide,
2-(4-hydroxy-3-methoxyphenyl)-2-hydroxy-N-heptylacetic acid amide,
2-(4-hydroxy-3-methoxyphenyl)-2-hydroxy-N-nonylacetic acid amide
and
2-(4-hydroxy-3-methoxyphenyl)-2-hydroxy-N-(7-methyl-1-octyl)acetic acid amide.

3. Process for the preparation of compounds according to claim 1 or 2, **characterised in that** a mandelic acid of the general formula (II) 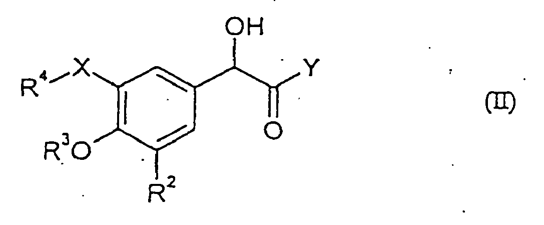 wherein
X, R², R³ and R⁴ have the meaning defined in claim 1
and
Y represents an activated leaving group,
or derivatives in which the OH groups are protected by protecting groups, is reacted with an alkylamine of the general formula (IIIa) 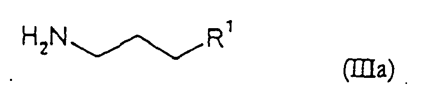 or with an alkylammonium salt of the general formula (IIIb) 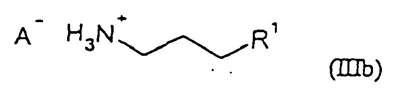 wherein R¹ has the meaning defined hereinbefore and A⁻ represents an inorganic or organic anion,
optionally in the presence of solvents and/or auxiliary bases, and the protecting groups of the OH groups are optionally removed.

4. Preparations used for nutrition, oral hygiene or consumed for pleasure, comprising mandelic acid alkylamides of the general formula (I) 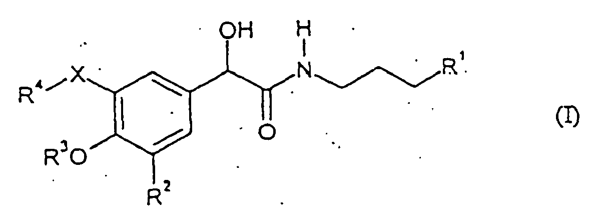 wherein
R¹ represents a linear or branched alkyl radical having from 1 to 20 carbon atoms or a linear or branched alkenyl radical having from 2 to 20 carbon atoms and
R² represents a hydrogen atom,
and
either
x represents a single bond,
R³ represents a lower alkyl radical or a lower alkenyl radical and
R⁴ represents hydrogen,
or
X represents an oxygen atom,
R³ represents hydrogen and
R⁴ represents a lower alkyl radical or a lower alkenyl radical,
or
X represents an oxygen atom,
R³ represents a lower alkyl radical or a lower alkenyl radical and
R⁴ represents hydrogen,
and the various stereoisomers thereof or mixtures thereof.

5. Preparations according to claim 4, comprising at least one further pungent-tasting or heat-generating substance.

6. Preparations according to claim 4, comprising at least one pungent-tasting plant extract.

7. Preparations according to claim 4, comprising at least one further pungent-tasting or heat-generating substance and at least one pungent-tasting plant extract.

8. Preparations according to at least one of claims 4 to 7 in the form of semi-finished products.

9. Preparations according to at least one of claims 4 to 8 in the form of fragrance, flavouring and taste-imparting compositions as well as spice mixtures.

10. Cosmetic or dermatological preparations comprising mandelic acid alkylamides of the general formula (I) 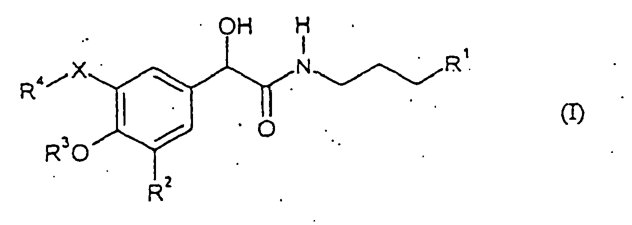 wherein X, R¹, R², R³ and R⁴ have the meaning defined in claim 4,
and the various stereoisomers thereof or mixtures thereof.

11. Use of mandelic acid alkylamides of the general formula (I) 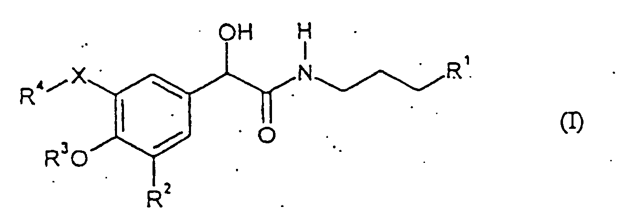 wherein X, R¹, R², R³ and R⁴ have the meaning defined in claim 4,
and the various stereoisomers thereof or mixtures thereof, as flavourings.

12. Use of
2-(4-methoxyphenyl)-2-hydroxy-N-heptylacetic acid amide,
2-(4-methoxyphenyl)-2-hydroxy-N-octylacetic acid amide,
2-(4-methoxyphenyl)-2-hydroxy-N-nonylacetic acid amide,
2-(3-hydroxy-4-methoxyphenyl)-2-hydroxy-N-heptylacetic acid amide,
2-(3-hydroxy-4-methoxyphenyl)-2-hydroxy-N-octylacetic acid amide,
2-(3-hydroxy-4-methoxyphenyl)-2-hydroxy-N-nonylacetic acid amide,
2-(4-hydroxy-3-methoxyphenyl)-2-hydroxy-N-heptylacetic acid amide,
2-(4-hydroxy-3-methoxyphenyl)-2-hydroxy-N-nonylacetic acid amide,
2-(4-hydroxy-3-methoxyphenyl)-2-hydroxy-N-octylacetic acid amide
and
2-(4-hydroxy-3-methoxyphenyl)-2-hydroxy-N-(7-methyl-1-octyl)acetic acid amide,
and the various stereoisomers thereof or mixtures thereof, as flavourings.

13. Use according to claim 11 or 12, wherein flavouring means pungent substance or flavouring having a heat-generating effect.

14. Use according to at least one of claims 11 to 13 in preparations used for nutrition or consumed for pleasure.

15. Use according to at least one of claims 11 to 14 in preparations used for oral hygiene.

16. Use of the mandelic acid alkylamides of formula (I), wherein X, R¹, R², R³ and R⁴ have the meaning defined in claim 4, in cosmetic or dermatological preparations.

## Revendications

1. Alkylamides d'acides mandéliques répondant à la formule générale (I) 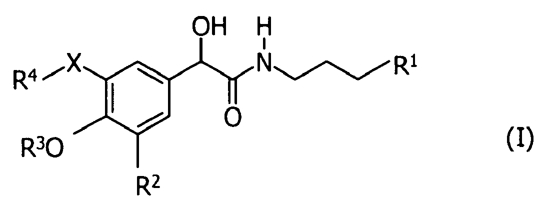 dans laquelle
R¹ représente un groupe alkyle linéaire ou ramifié contenant 1 à 20 atomes de carbone ou un groupe alcényle linéaire ou ramifié contenant 2 à 20 atomes de carbone,
R² représente un atome d'hydrogène,
et
ou bien
X représente une liaison simple,
R³ représente un groupe alkyle inférieur ou alcényle inférieur, et
R⁴ représente l'hydrogène,
ou bien
X représente un atome d'oxygène,
R³ représente l'hydrogène et
R⁴ représente un groupe alkyle inférieur ou alcényle inférieur,
ou bien
X représente un atome d'oxygène,
R³ représente un groupe alkyle inférieur ou alcényle inférieur et
R⁴ représente l'hydrogène,
et leurs divers stéréoisomères ou leurs mélanges, à l'exception des cas pour lesquels X représente un atome d'oxygène, R¹ un groupe 1-pentyle, R² et R³ l'hydrogène et R⁴ un groupe méthyle.

2. Le 2-(4-méthoxyphényl)-2-hydroxy-N-heptylacétamide,
le 2-(4-méthoxyphényl)-2-hydroxy-N-octylacétamide,
le 2-(4-méthoxyphényl)-2-hydroxy-N-nonylacétamide,
le 2-(3-hydroxy-4-méthoxyphényl)-2-hydroxy-N-heptylacétamide,
le 2-(3-hydroxy-4-méthoxyphényl)-2-hydroxy-N-octylacétamide,
le 2-(3-hydroxy-4-méthoxyphényl)-2-hydroxy-N-nonylacétamide,
le 2-(4-hydroxy-3-méthoxyphényl)-2-hydroxy-N-heptylacétamide,
le 2-(4-hydroxy-3-méthoxyphényl)-2-hydroxy-N-nonylacétamide,
et
le 2-(4-hydroxy-3-méthoxyphényl)-2-hydroxy-N-(7-méthyl-1-octyl)-acétamide,

3. Procédé pour la préparation des composés selon la revendication 1 ou 2, **caractérisé en ce que** l'on fait réagir un acide mandélique de formule générale II 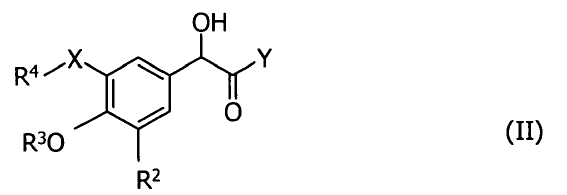 dans laquelle
X, R², R³ et R⁴ ont les significations indiquées dans la revendication 1,
et
Y représente un groupe éliminable activé,
ou des dérivés de cet acide dont les groupes OH sont protégés par des groupes appropriés,
avec une alkylamine de formule générale (IIIa) 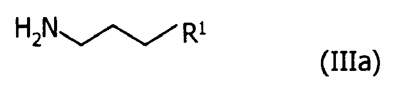 ou avec un sel d'alkylammonium de formule générale (IIIb) 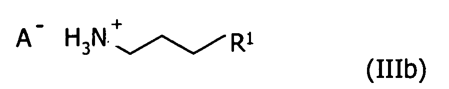 dans lesquelles R¹ a les significations indiquées ci-dessus et A⁻ représente un anion organique ou minéral,
le cas échéant en présence de solvants et/ou de bases auxiliaires, et le cas échéant on élimine les groupes protecteurs des groupes OH.

4. Compositions alimentaires, pour l'hygiène buccale ou de consommation, contenant des alkylamides d'acides mandéliques de formule générale (I) 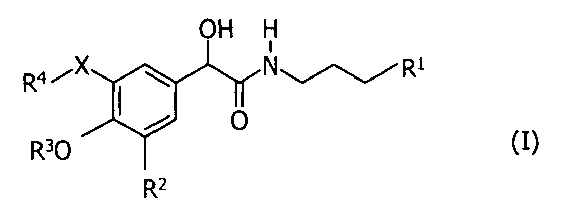 dans laquelle
R¹ représente un groupe alkyle linéaire ou ramifié contenant 1 à 20 atomes de carbone ou un groupe alcényle linéaire ou ramifié contenant 2 à 20 atomes de carbone,
R² représente un atome d'hydrogène,
et
ou bien
X représente une liaison simple,
R³ représente un groupe alkyle inférieur ou alcényle inférieur, et
R⁴ représente l'hydrogène,
ou bien
X représente un atome d'oxygène,
R³ représente l'hydrogène et
R⁴ représente un groupe alkyle inférieur ou alcényle inférieur,
ou bien
X représente un atome d'oxygène,
R³ représente un groupe alkyle inférieur ou alcényle inférieur et
R⁴ représente l'hydrogène,
et leurs divers stéréoisomères ou leurs mélanges.

5. Compositions selon la revendication 4 contenant au moins une autre substance à goût piquant ou donnant une sensation de chaleur.

6. Compositions selon la revendication 4, contenant au moins un extrait végétal à goût piquant.

7. Compositions selon la revendication 4, contenant au moins une autre substance à goût piquant ou donnant une sensation de chaleur et au moins un extrait végétal à goût piquant.

8. Compositions selon au moins une des revendications 4 à 7, à l'état de produits semi-finis.

9. Compositions selon au moins une des revendications 4 à 8, à l'état de compositions odorantes, aromatiques et gustatives ou de mélanges d'épices.

10. Compositions cosmétiques ou dermatologiques, contenant des alkylamides d'acides mandéliques de formule générale (I) 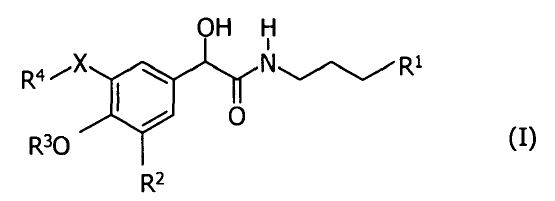 dans laquelle X, R¹, R², R³ et R⁴ ont les significations indiquées dans la revendication 4,
et leurs divers stéréoisomères ou leurs mélanges.

11. Utilisation des alkylamides d'acides mandéliques de formule générale (I) 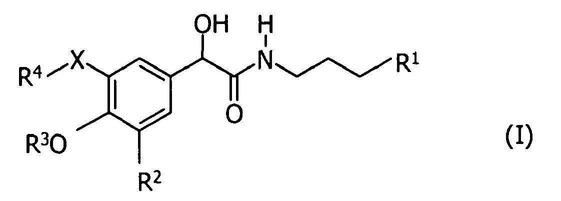 dans laquelle X, R¹, R², R³ et R⁴ ont les significations indiquées dans la revendication 4,
et de leurs divers stéréoisomères ou de leurs mélanges, en tant que substances aromatiques.

12. Utilisation du
2-(4-méthoxyphényl)-2-hydroxy-N-heptylacétamide,
2-(4-méthoxyphényl)-2-hydroxy-N-octylacétamide,
2-(4-méthoxyphényl)-2-hydroxy-N-nonylacétamide,
2-(3-hydroxy-4-méthoxyphényl)-2-hydroxy-N-heptylacétamide,
2-(3-hydroxy-4-méthoxyphényl)-2-hydroxy-N-octylacétamide,
2-(3-hydroxy-4-méthoxyphényl)-2-hydroxy-N-nonylacétamide,
2-(4-hydroxy-3-méthoxyphényl)-2-hydroxy-N-heptylacétamide,
2-(4-hydroxy-3-méthoxyphényl)-2-hydroxy-N-nonylacétamide,
2-(4-hydroxy-3-méthoxyphényl)-2-hydroxy-N-octylacétamide,
et
2-(4-hydroxy-3-méthoxyphényl)-2-hydroxy-N-(7-méthyl-1-octyl)acétamide,
et de leurs divers stéréoisomères ou de leurs mélanges en tant que substances aromatiques.

13. Utilisation selon la revendication 11 ou 12, pour laquelle substance aromatique signifie substance piquante ou substance aromatique donnant une sensation de chaleur.

14. Utilisation selon au moins une des revendications 11 à 13 dans les compositions alimentaires ou de consommation.

15. Utilisation selon au moins une des revendications 11 à 14 dans des compositions pour l'hygiène buccale.

16. Utilisation des alkylamides d'acides mandéliques de formule (I) dans laquelle X, R¹, R², R³ et R⁴ ont les significations indiquées dans la revendication 4, dans des compositions cosmétiques ou dermatologiques.
